# DEMANDE DE BREVET EUROPEEN

(11) **EP 0 740 960 A1**
(43) Date de publication de la demande: **06.11.1996**
(21) Numéro de dépôt: 96106415.1
(22) Date de dépôt: 24.04.1996
(51) Int. Cl.: B01L 11/00, G01N 27/28, B65D 83/08

(54) **Appareil perfectionné destiné à la distribution de zones successives d'une bande consommable**

(30) Priorité: 02.05.1995 FR 9505224
(71) Demandeur: ASULAB S.A., CH-2501 Bienne (CH)
(72) Inventeur: Jaeger,Gérard, 1807 Blonay (CH)
(74) Mandataire: Patry, Didier Marcel Pierre

(57) **Abrégé**

Cet appareil fonctionne avec des bandes consommables qui y sont introduites par un utilisateur.

Il comprend un couloir (29) pour les bandes (34) dans lequel peut circuler un curseur (35) auquel chaque bande peut être accouplée. Des moyens d'encliquetage (54,57,62a à 65) associé à un organe d'entraînement solidaire du curseur sont agencés pour faire avancer ce curseur pas à pas en direction de l'entrée (28) du couloir (29) depuis n'importe quelle position dans laquelle le curseur est susceptible de se trouver, et ceci par un entraînement direct du curseur. De ce fait, il est toujours possible de ramener le curseur dans une position avancée, proche de ladite entrée.

Application notamment à des dispositifs de distribution d'objets tels que des cachets de médicaments ou à des dispositifs de mesure du taux de glucose dans le sang.

## Description

La présente invention est relative à un appareil distributeur nécessitant pour fonctionner l'emploi d'éléments consommables se présentant sous la forme de bande, chacune de ces bandes comprenant dans le sens longitudinal plusieurs zones d'utilisation successives destinées à être séparées de la bande dès après leur utilisation.

Plus particulièrement, l'invention concerne un tel appareil formant un dispositif de mesure permettant de mesurer un paramètre d'une substance déposée sur des zones successives d'une bande formant capteur de mesure consommable, appelé aussi capteur multizone.

Un tel dispositif de mesure peut avantageusement servir à la mesure du taux de glucose dans le sang, à l'usage des diabétiques. Il a été décrit sous différents aspects dans plusieurs demandes de brevet français déposées au nom de la Demanderesse et parmi lesquelles l'on peut citer le FR 92 01 331 pour ce qui concerne le procédé de mesure électrochimique utilisé, le FR 93 11 316 pour ce qui concerne un dispositif de coupe permettant de séparer de la bande consommable les zones de mesure utilisées, le FR 93 11 317 pour ce qui concerne un dispositif de connexion électrique permettant de relier la bande captrice à un circuit électronique de mesure destiné à élaborer le résultat de la mesure sous une forme perceptible à un utilisateur, et enfin le FR 93 11 319 qui concerne plus spécifiquement un dispositif d'éjection du dernier tronçon de la bande captrice, lorsque toutes les zones de mesure ont été utilisées.

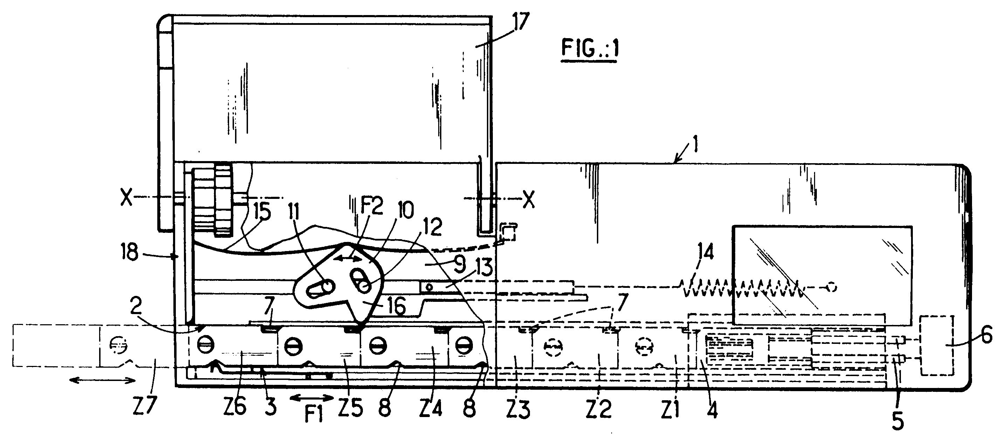

La figure 1 des dessins annexés montre un dispositif de mesure dans lequel on retrouve, sous une forme sommaire, un exemple de réalisation des perfectionnements qui ont fait l'objet des demandes de brevet susindiquées, étant entendu que pour en trouver une description détaillée, l'on peut se référer aux textes desdites demandes de brevet.

Ainsi, le dispositif de mesure comporte un boîtier 1, de forme générale allongée et d'une taille telle qu'il puisse facilement être tenu dans le creux de la main d'un adulte. Ce boîtier définit un couloir 2 de circulation orienté longitudinalement dans le boîtier 1 et destiné à la circulation d'une bande captrice 3 (double flèche F1), ainsi que d'un curseur 4. Ce dernier est chargé de transmettre le signal électrique provenant de la bande captrice 3 à des lignes électriques 5 s'étendant le long du couloir 2. Ces lignes sont à leur tour connectées à un circuit électronique 6 destiné à exploiter convenablement ce signal électrique pour le rendre intelligible à un utilisateur au moyen d'un dispositif d'affichage prévu également dans le boîtier 1.

Dans l'exemple de la figure 1, la bande captrice 3 est conformée spécifiquement à la mesure du taux de glucose dans le sang. Une description détaillée d'une telle bande peut être trouvée dans la première demande de brevet précitée. Il suffit de retenir ici qu'elle comporte plusieurs zones de mesure, sept en l'occurrence, référencées Z1 à Z7 dont la zone Z7 représentée en pointillés est supposée avoir déjà été utilisée et tronçonnée de la bande 3.

On remarquera également que la bande captrice comprend pour toutes les zones Z1 à Z7 un cran d'avancement 7 sur l'un de ses bords longitudinaux ainsi que pour toutes les zones, sauf la zone Z7, une encoche de positionnement 8 située sur l'autre bord de la bande.

Les crans d'avancement 7 coopèrent avec un mécanisme 9 d'avance longitudinal de la bande 3 le long de son couloir 2. Ce dernier comprend un bouton d'actionnement (non visible sur la figure 1) monté en coulissement longitudinal dans le boîtier c'est-à-dire comme vu sur la figure 1, au dessus du couloir 2.

Le bouton d'actionnement coopère avec un cliquet basculant 10 guidé sur deux tétons 11 et 12 solidaires d'une réglette 13. Celle-ci est couplée au bouton d'actionnement et montée coulissante dans le boîtier 1 dans la direction de la flèche F1 à l'encontre de l'action d'un ressort de rappel 14 ancré sur le boîtier 1. Le cliquet basculant 10 est maintenu en position non active (représentée sur la figure 1) par un ressort à lame 15 et comporte un bec d'actionnement 16 destiné à coopérer avec les crans d'avancement 7 de la bande captrice 3.

Dès lors, on comprend que le bouton d'actionnement étant activé en va et vient, la bande captrice avance de la longueur d'une zone de mesure de la bande, le mécanisme d'avance faisant basculer le cliquet 10 en va et vient (selon la flèche F2) pour d'abord pousser la bande hors du couloir d'un pas puis revenir vers la position inactive telle que représentée, en se dégageant de la bande.

Le dispositif comprend aussi un couvercle 17 monté rotatif sur le boîtier autour d'un axe X-X et couplé mécaniquement à un mécanisme de coupe 18 permettant de tronçonner la zone de la bande consommable que l'on vient d'utiliser, et ce par un simple mouvement de fermeture du couvercle 17.

Il est encore à noter que le curseur 4 coulisse librement dans le couloir 2 et est repoussé vers l'arrière lors de l'insertion de la bande captrice consommable 3. Celle-ci y est couplée mécaniquement lors de cette insertion lorsque le curseur 4 vient buter contre le fond du couloir 2. A cet effet, le curseur porte un organe de couplage élastique cédant sous la force d'insertion exercée sur la bande 3 par l'utilisateur, pour s'accrocher à cette bande dès que la force d'insertion est relâchée.

Des tests d'utilisation du dispositif de mesure qui vient d'être décrit, ont montré qu'il fonctionne convenablement et sans difficultés tant que la bande captrice est consommée au fur et à mesure et qu'il ne se produit aucun incident tel que par exemple la détérioration d'un de ses crans d'avancement.

Il se peut également que l'utilisateur, par distraction, engage une nouvelle bande dans le boîtier alors qu'il y en a déjà une dans le couloir 2 non encore consommée en entier. L'utilisateur repousse alors l'ancien morceau de bande , avec le curseur qui y est couplé, dans le fond du couloir. Cependant, l'utilisateur, s'apercevant de son erreur, enlèvera la bande neuve sans toutefois pouvoir ramener vers l'avant ni l'ancien morceau, ni le curseur.

L'invention a pour but d'apporter une solution au problème évoqué ci-dessus.

L'invention a donc pour objet un appareil distributeur nécessitant pour fonctionner l'emploi d'éléments consommables se présentant sous la forme de bandes, chacune de ces bandes comprenant dans le sens longitudinal plusieurs zones d'utilisation successives destinées à être séparées de la bande dès après leur utilisation, ledit appareil comprenant:
- un boîtier définissant un couloir de circulation pour lesdites bandes, ledit couloir présentant, compte tenu du sens d'introduction desdites bandes, une extrémité amont et une extrémité aval,
- un mécanisme d'avance pour faire sortir ladite bande du couloir de circulation selon un mouvement pas-à-pas après son introduction dans celui-ci par un utilisateur,
- une unité coulissante montée mobile dans ledit couloir, couplée fonctionnellement audit mécanisme d'avance et destinée à être accouplée à l'extrémité aval de ladite bande lors de son introduction par l'utilisateur, et
- des moyens de couplage portés par ladite unité coulissante et agencés pour assurer le couplage entre celle-ci et ladite bande,
   ledit appareil distributeur étant caractérisé en ce que
- ladite unité coulissante comprend un curseur monté coulissant dans ledit couloir de circulation et muni d'un organe d'entraînement,
- ledit mécanisme d'avance comprend des moyens d'encliquetage destinés à coopérer avec ledit organe d'entraînement pour assurer ledit couplage et permettre l'avance pas-à-pas dudit curseur, et
- lesdits moyens d'encliquetage sont couplés à un bouton d'actionnement monté mobile dans ledit boîtier pour permettre la commande dudit mouvement pas-à-pas.

Grâce à ces caractéristiques, la bande consommable est entraînée selon le mouvement pas-à-pas par l'intermédiaire de l'unité coulissante à laquelle elle est couplée lors de son insertion dans le couloir de circulation, unité coulissante qui, elle, est en prise avec les moyens d'encliquetage prévus dans le boîtier. Le mouvement pas-à-pas devient ainsi indépendant de la présence ou de l'absence de la bande consommable. Celle-ci peut donc être incomplète, voire détériorée et malgré cela être toujours évacuée de l'appareil. Par ailleurs, la bande consommable n'a pas à être profilée elle-même pour coopérer avec les moyens d'avance. Elle peut donc être plus simple et dans la détermination de sa largeur, on n'a plus à tenir compte de la présence de crans d'entraînement ou analogues.

D'autres caractéristiques et avantages de l'invention apparaîtront au cours de la description qui va suivre, donnée uniquement à titre d'exemple et faite en se référant aux dessins annexés sur lesquels:
- la figure 1, déjà décrite, est une vue schématique en plan, avec arrachement partiel, d'un appareil distributeur conforme aux demandes de brevet précitées;
- les figures 2A, 2B et 2C sont trois vues extérieures, selon trois plans orthogonaux, d'un appareil distributeur selon l'invention;
- la figure 3 est une vue en plan d'un appareil distributeur selon l'invention, la partie supérieure de son boîtier étant supposée enlevée et certaines parties en étant représentées avec arrachement partiel;
- la figure 4 est une vue en plan, à échelle agrandie, de l'appareil selon l'invention, pour en illustrer les caractéristiques essentielles, et notamment la configuration du mécanisme d'avance;
- les figures 5 et 5A sont des vues en coupe prises respectivement selon les lignes V-V et VA-VA de la figure 4;
- la figure 6 montre en quatre vues, la cinématique des moyens d'avance utilisés dans l'appareil suivant l'invention.

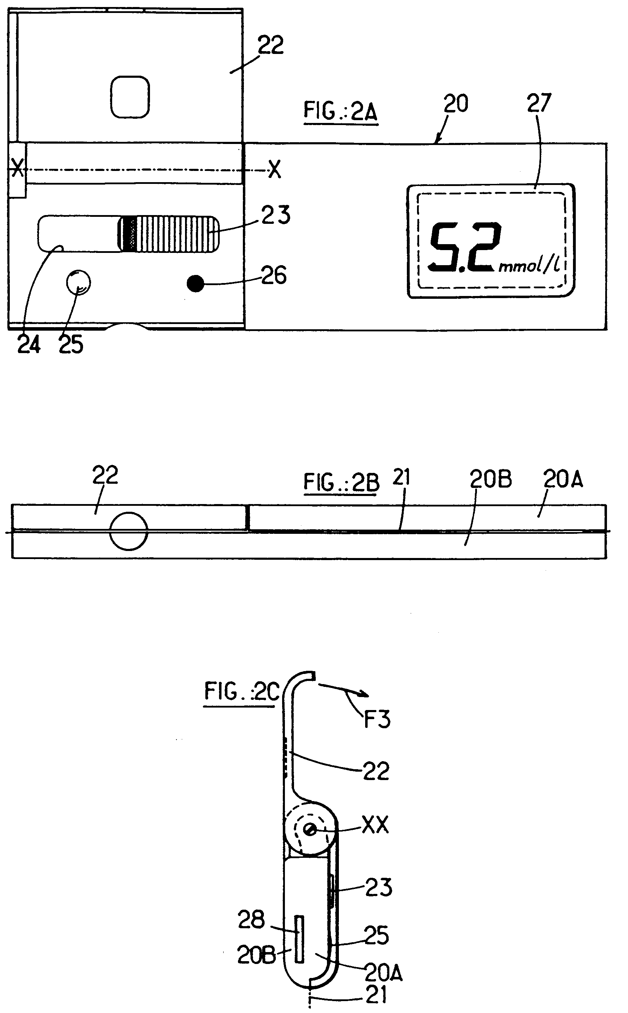

Les figures 2 à 6 représentent un exemple préféré de réalisation de l'appareil distributeur suivant l'invention dans son application à un dispositif permettant de mesurer le taux de glucose dans le sang à l'usage des diabétiques, par exemple. Cependant, l'invention n'est pas limitée à cette application spécifique. En effet, elle peut être mise en oeuvre, dans toutes sortes d'autres cas dans lesquels il est souhaitable d'utiliser des bandes consommables sur lesquelles des zones adjacentes définissent une aire d'utilisation spécifique, ces zones après avoir été utilisées pouvant, voire devant (pour des raisons médicales par exemple) être ôtées de la bande pour laisser la place à une zone suivante. Par exemple, dans le domaine médicale, un tel appareil pourrait être utilisé pour la distribution d'une série de cachets ou pilules de médicaments devant, selon la posologie prescrite par le médecin, être pris dans un ordre strict défini à l'avance. Toutefois, l'invention ne se limite pas davantage aux seules applications dans le domaine médical.

L'appareil distributeur selon l'invention représenté aux figures 2 à 6 comporte un boîtier 20 fait en deux demi-coquilles 20A et 20B, en matière plastique moulée de préférence, et assemblées l'une à l'autre selon un plan de joint 21. Dans l'application décrite, le boîtier 20 a une forme allongée et présente des dimensions telles qu'il puisse facilement être tenu dans le creux de la main d'un adulte.

L'appareil comporte un couvercle 22 s'étendant sur une partie de sa longueur et articulé sur le boîtier 20 autour d'un axe longitudinal X-X.

Sur les figures 2A et 2C, on a représenté le couvercle 22 en position ouverte, de sorte que l'on aperçoit un bouton 23 coulissant dans un guide 24 ménagé dans la demi-coquille supérieure 20A du boîtier 20. Une petite loupe 25 permet d'observer une partie de la bande consommable (non représentée ici) glissée dans l'appareil. Un poussoir 26 est destiné à rappeler des mesures effectuées antérieurement et stockées dans la mémoire de l'appareil, pour l'affichage sur un écran 27 servant à permettre également la lecture immédiate des résultats de la mesure qui vient d'être faite.

Il est à noter que l'appareil est mis en état de marche, dès que le bouton 23 est actionné et que son alimentation (par une pile incorporée de préférence) est coupée après l'écoulement d'un délai prédéterminé (60 secondes, par exemple) s'écoulant à partir du moment où toute intervention sur l'appareil par l'utilisateur a cessé.

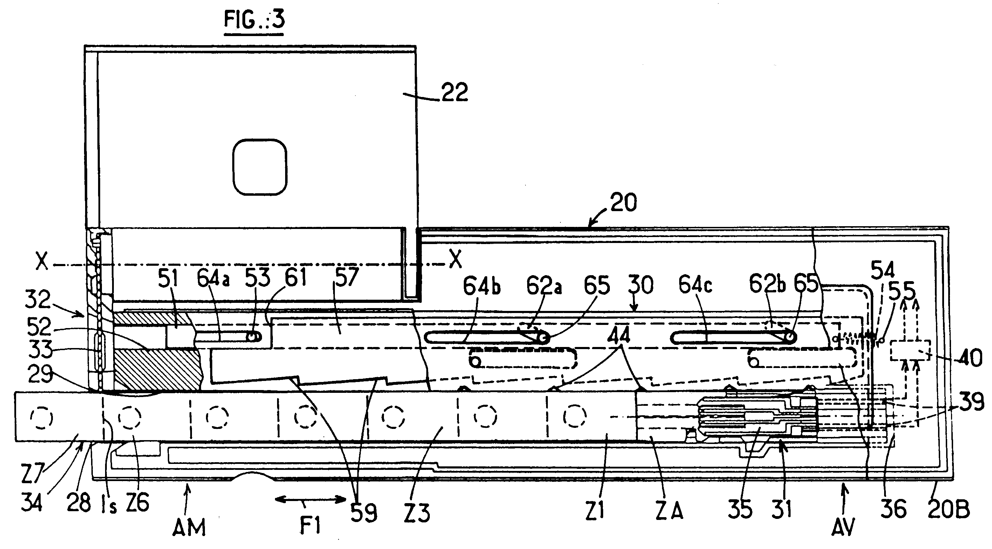

Du côté du couvercle 22, sur le chant frontal latéral du boîtier 20, l'appareil présente une ouverture 28 qui est l'embouchure d'un couloir de circulation 29 (figure 3) des bandes consommables.

La figure 3 montre l'appareil distributeur selon l'invention, alors que l'on en a ôté la demi-coquille supérieure 20A. De la sorte, on peut observer plusieurs unités fonctionnelles et notamment:
- un mécanisme d'avance 30 qui constitue plus particulièrement l'objet de la présente invention et qui permet d'avancer pas-à-pas une bande consommable dans le couloir 29 sous l'action répétée du bouton coulissant 23;
- une unité coulissante 31, destinée à être accouplée à une bande consommable et coulissant dans le couloir 29;
- une unité de coupe 32 située le long du chant d'extrémité de l'appareil et comprenant un couteau 33 monté coulissant dans une direction perpendiculaire à l'axe X-X; et
- une bande consommable 34 présentant sept zones de mesure Z1 à Z7 et une zone ZA formant amorce et permettant son couplage à l'unité coulissante 31.

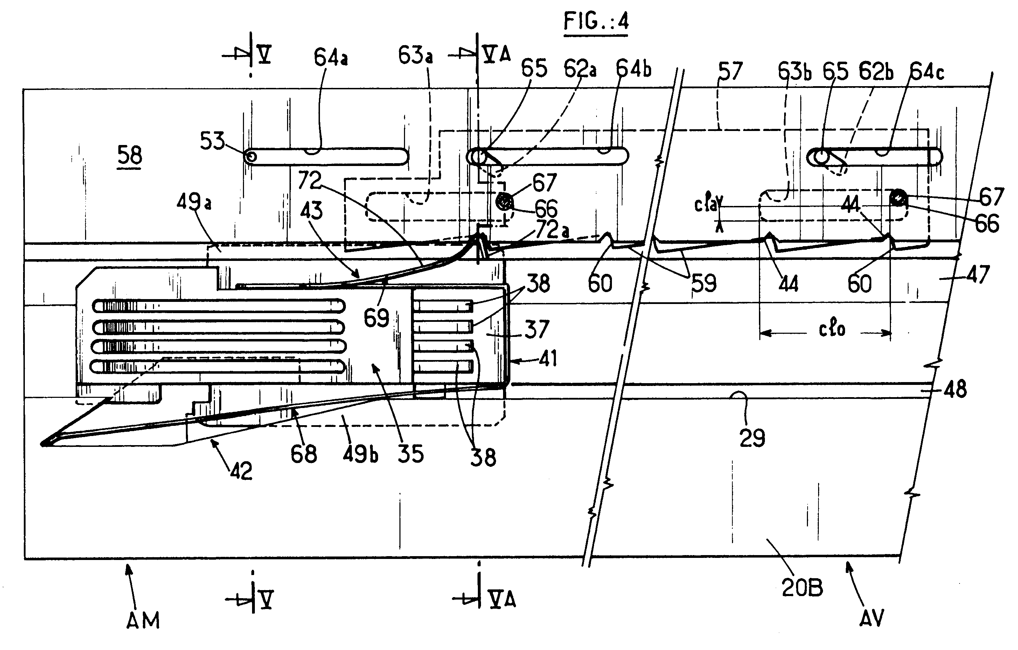

On va maintenant se référer aux figures 4 à 6 pour décrire plus spécifiquement les caractéristiques de la présente invention qui ont trait au mécanisme d'avance 30.

L'unité coulissante 31 comprend un curseur 35 pouvant circuler dans le couloir 29 qui est obturé à son extrémité 36 opposée à l'embouchure 28. Dans ce qui va suivre, on appellera cette extrémité, "extrémité aval" AV en considérant le mouvement d'introduction de la bande consommable 34 dans le couloir 29. De même, l'extrémité opposée, près de l'embouchure 28 sera appelée "extrémité amont" AM. Ces termes seront également utilisés pour la bande consommable, son extrémité aval étant donc située près de la zone d'amorce ZA.

Le curseur 35 comprend un corps de curseur 37 (figure 4) portant des moyens électriques de transmission 38 chargés d'assurer le prélèvement des signaux électriques de la bande 34 et la transmission de ces signaux à des pistes de contact 39 qui courent le long de la paroi supérieure du couloir 29. Selon une variante cette transmission peut être effectuée au moyen d'un câble flexible plat qui se développe dans le couloir 29. Les pistes 39 sont connectées à un circuit électronique de traitement 40 (figure 3) relié à son tour à l'écran d'affichage 27.

Le corps 37 porte également des moyens élastiques 41 de blocage et de couplage qui coopèrent avec des moyens 42 formant butée venus de moulage dans la demi-coquille inférieure 20B du boîtier 20.

Le corps 37 porte en outre des moyens 43 d'entraînement et de positionnement destinés à coopérer avec les moyens d'avance 30 et avec des crans d'indexage 44 prévus le long du couloir 29 et définissant respectivement les positions de mesure de la bande 34.

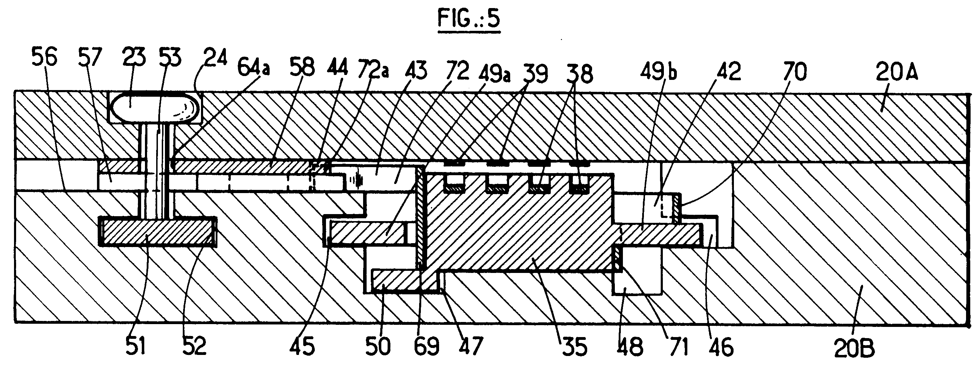

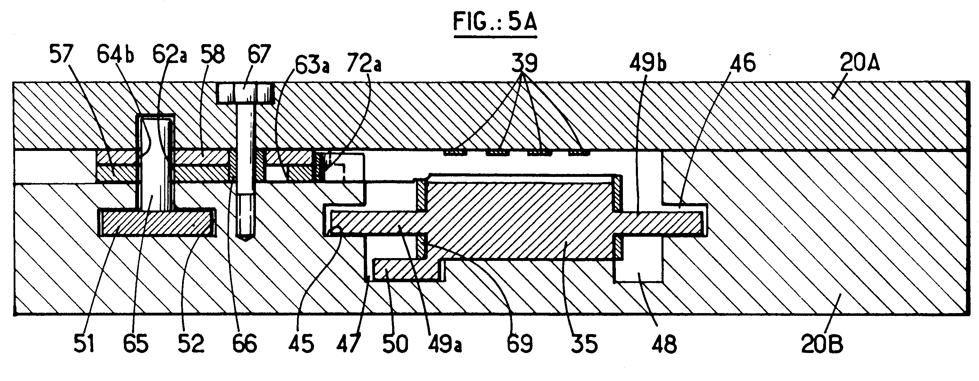

Sur les figures 4, 5 et 5A en particulier, on voit que le couloir de guidage 29 présente deux rainures longitudinales latérales 45, 46 et deux rainures longitudinales 47 et 48 ménagées dans son fond. Le corps 37 est de forme générale parallélépipédique d'où font saillie deux nervures latérales 49a et 49b, s'ajustant respectivement dans les rainures latérales 45 et 46 et une nervure inférieure 50 qui s'ajuste dans la rainure 47 prévue dans le fond du couloir 29.

Selon l'invention, les moyens d'avance 30 comprennent une réglette 51 de section rectangulaire montée mobile dans une rainure longitudinale 52 à section en forme de T inversé et prévue dans la demi-coquille 20B. Cette réglette 51 est solidaire d'une première tige 53 faisant saillie hors de la rainure 52 et passant à travers la demi-coquille 20A pour être engagée à force dans le bouton coulissant 23.

La réglette 51 est sollicitée en permanence dans le sens AV sous l'action d'un ressort de rappel 54 logé, côté aval, dans la rainure 52 (figure 3). Ce ressort 54 est accroché par l'une de ces extrémités à la réglette 51, tandis que son extrémité opposée est fixée sur un petit ergot 55 venu de moulage avec la demi-coquille 20B et situé à l'extrémité aval de la rainure 52.

Une partie de la surface supérieure de la demi-coquille 20B forme une surface de glissement 56 s'étendant le long du couloir 29. Elle sert d'appui à une barre d'encliquetage 57 destinée à coulisser en va-et-vient selon la direction de la double flèche F1.

Cette barre d'encliquetage 57 est prise entre la surface de glissement 56 et une plaque de guidage allongée 58 disposée au-dessus de la barre d'encliquetage 57 et rendue solidaire de la demi-coquille 20B. Il est à noter que les crans de positionnement 44 pour l'unité coulissante 31 sont prévus dans le bord de cette plaque de guidage.

Le bord de la barre d'encliquetage 57 longeant le couloir 29 présente une denture 59 à dents de scie, le flanc court 60 de chaque dent étant situé du côté aval AV.

Le nombre des dents de cette denture est égal au nombre de zones prévues sur une bande consommable 34 ayant la longueur maximale acceptable par l'appareil. Il est à noter au passage que celui-ci peut accepter des bandes consommables de toutes les longueurs entre cette longueur maximale et celle correspondant à un nombre minimale de zones qu'il est raisonnablement utile de prévoir sur la bande consommable (En théorie, on pourrait ne prévoir qu'une seule zone sur la bande).

La barre d'encliquetage 57 présente également:
- une découpe avant 61 (figure 3) s'étendant dans le sens de la longueur de la barre, au dessus de l'ouverture de la rainure en forme de T 52 pour notamment permettre le mouvement de la tige 53, lorsque le bouton 23 est actionné;
- deux lumières 62a et 62b en forme de boutonnière placées obliquement par rapport à la direction longitudinale générale de la barre, c'est-à-dire à la direction de la double flèche F1; et
- deux découpes 63a et 63b de forme rectangulaire servant au guidage de la barre d'encliquetage 57.

La plaque de guidage 58 présente trois lumières allongées 64a, 64b et 64c, alignées les unes sur les autres dans le sens longitudinal, donc selon la direction de la double flèche F1.

La première de ces lumières 64a est traversée par la tige 53 déjà mentionnée. La seconde 64b et la troisième 64c sont traversées par des tiges d'entraînement 65 passant respectivement également dans les boutonnières 62a et 62b de la barre d'encliquetage 57. Elles sont fixées sur la réglette 51 et se déplacent donc de concert avec le bouton 23.

Les découpes 63a et 63b sont traversées par des douilles 66 formant organe suiveur, servant d'entretoises et à travers lesquelles passent également des vis de serrage 67 vissées dans la demi-coquille 20B.

La longueur des découpes 63a et 63b définit la course longitudinale clo de la barre d'encliquetage, tandis que leur largeur en détermine la course latérale cla (figure 4). De cette manière, les découpes 63a et 63b en coopération avec les douilles 66 assurent la définition du mouvement de la barre 57 selon un trajet orbital de tracé rectangulaire.

Les moyens élastiques 41 de blocage et de couplage et les moyens 43 d'entraînement et de positionnement sont de préférence réalisés dans une même lame élastique. Celle-ci présente une forme en U et peut par exemple être surmoulée par de la matière plastique au moment du formage du curseur 35. L'âme du U est appliquée contre la face aval du curseur 35, tandis que ses branches 68 et 69 s'étendent vers l'embouchure 28 du couloir 29 en longeant respectivement les flancs latéraux du curseur 35.

La branche 68 comporte deux ressorts 70 et 71 destinés à assurer le blocage du curseur 35 dans la position amont AM et à le libérer dès l'insertion d'une bande consommable dans le couloir 29. Cet aspect de l'appareil ne faisant pas partie de l'invention, on ne le décrira pas en détail ici.

Par contre, la branche 69 joue un rôle primordial dans la mise en oeuvre de l'invention. Elle constitue en fait les moyens 43 d'entraînement et de positionnement déjà mentionnés. Elle comprend un ressort 72 s'étendant vers l'aval et se terminant par un crochet 72a. Celui-ci est destiné à coopérer avec les crans 44 et également avec la denture 59 de la barre d'encliquetage 57.

On notera que les rives de la bande consommable ne comportent aucune encoche ou autre élément nécessaire pour l'entraînement de la bande. En effet, grâce aux caractéristiques particulières de l'invention, cet entraînement est assuré exclusivement par l'intermédiaire du curseur 35 sur lequel agit le mécanisme d'avance 31 que l'on vient de décrire. La forme de la bande consommable est donc des plus simples.

Sur la figure 3, on a représenté l'appareil, alors que l'utilisateur vient d'y insérer une bande consommable. Toutefois, la bande n'est pas encore tout à fait parvenue au bout du couloir 29. Pour cela, la ligne de séparation ls (fictive) entre les zones Z7 et Z6 doit encore être placée devant le tranchant de l'unité de coupe 32. Le mécanisme d'avance est au repos; la barre d'encliquetage 57 est donc dans sa position aval, sa denture 59 étant effacée hors du couloir 29.

En revanche, sur la figure 4, le curseur 35 se trouve dans la position extrême amont dans le couloir 29 sans qu'une bande ne soit présente dans l'appareil. Cependant, on suppose dans cette figure que l'utilisateur vient de manoeuvrer le bouton 23 pour la dernière fois, que la zone d'amorce ZA a déjà été éjectée de l'appareil, mais que l'utilisateur n'a pas encore lâché le bouton 23. De ce fait, dans cette configuration, la barre d'encliquetage 57 se trouve encore dans sa position AM, le ressort de rappel 54 est tendu et le petit flanc 60 de la dent la plus amont de la denture 59 est encore en prise avec le crochet 72a du ressort 72. Par ailleurs, la tige 53 engagée dans la bouton 23 se trouve à l'extrémité aval de la rainure 64a de la plaque de guidage 58.

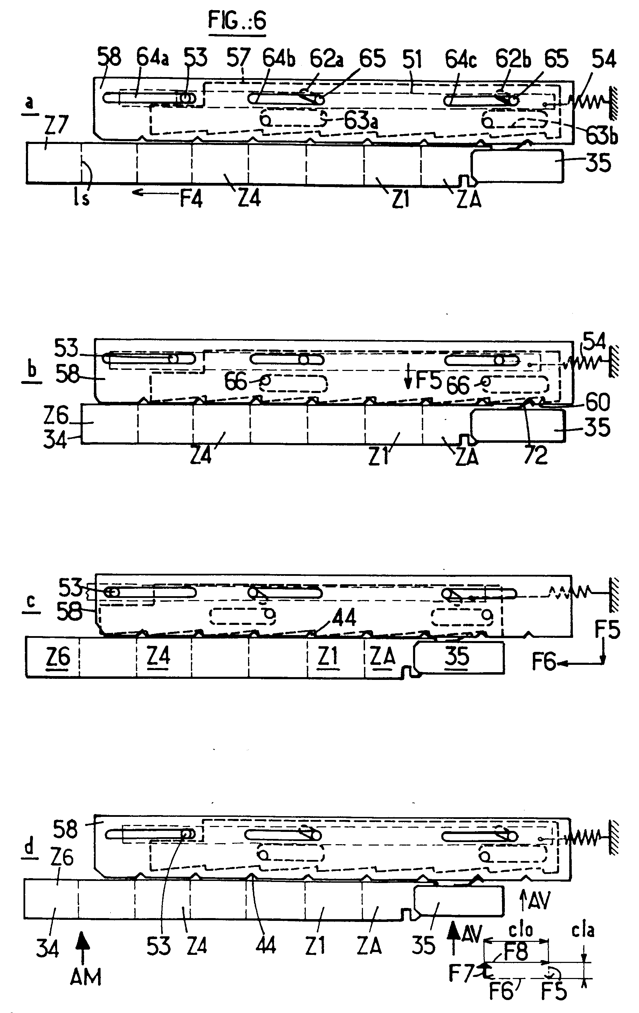

Les quatre vues a à d de la figure 6 représentent les positions principales des composants de l'appareil selon l'invention au cours d'un cycle complet d'avance de la bande consommable.

Plus précisément, sur la figure 6a et par rapport à la figure 3, la bande consommable 34 se trouve complètement insérée dans l'appareil, le curseur 35 étant donc à fond de course à l'extrémité aval du couloir 29.

Le ressort 54 maintient la barre d'encliquetage 57 dans la position aval et en raison de l'obliquité des lumières 62a et 62b, cette barre occupe la position latérale extérieure par rapport au couloir 29.

La zone Z7 de la bande qui fait alors saillie hors de l'appareil peut donc être utilisée pour une mesure du taux de glucose. L'utilisateur ayant terminée cette mesure, il ferme le couvercle 22 coupant en même temps la zone Z7 le long de la ligne de séparation ls avec la zone Z6.

L'utilisateur, lors d'une nouvelle utilisation de l'appareil, doit alors en faire sortir la zone Z6. Pour ce faire, il ouvre le couvercle 22, puis actionne le bouton 23 dans le sens de la flèche F4 (figure 6b). Il en résulte d'abord la configuration des composants représentée sur cette figure, configuration qui n'est que fugitive, mais qui montre que la barre d'encliquetage 57 se déplace d'abord latéralement vers l'intérieur dans le sens de la flèche F5 sur la course cla, de nouveau grâce à l'obliquité des lumières 62a et 62b. La denture 59 est alors placée dans la région d'action du ressort 72.

Le bouton 23 continuant à être avancé, le flanc 60 le plus en aval de la denture 59 va ensuite venir en contact du crochet 72a du ressort 72, à l'encontre de la force du ressort de rappel 54, puis ce crochet exerce une force d'entraînement sur le curseur 35. Celui-ci se déplace donc vers l'extrémité amont AM du couloir 29 en poussant la bande consommable 34 devant lui (sens de la flèche F6).

Le mouvement dans ce sens s'arrête, lorsque la course clo est accomplie, c'est-à-dire quand les bords aval des découpes 63a et 63b viennent en contact des douilles 66 engagées autour des vis 67. Cette course correspondant exactement à la longueur d'une zone sur la bande consommable 34, la zone Z6 se trouvera donc hors de l'appareil.

L'utilisateur n'a plus alors qu'à lâcher le bouton 23 ce qui ramène la barre d'encliquetage 57 vers sa position de départ sous l'action du ressort 54 et selon le trajet symbolisé par les flèches F7 et F8 sur la figure 6d. Par ailleurs, le crochet 72a du ressort 72 dont est équipé le curseur 37 s'étant placé dans le cran d'indexage 44 correspondant à la zone Z6 de la bande consommable, celle-ci reste stable dans sa nouvelle position.

On voit donc que ce cycle de fonctionnement implique un mouvement orbital de la barre d'encliquetage selon un tracé qui présente une courbe fermée rectangulaire selon les flèches F5 à F8 représentées sur la figure 6.

Bien entendu, ce cycle peut se répéter pour toutes les zones de mesure de la bande consommable 34, jusqu'à ce qu'il ne reste plus que la zone d'amorce ZA dans l'appareil. Cependant, dans la position correspondante du curseur 35, cette zone est découplée de celui-ci et l'utilisateur peut donc l'ôter facilement de l'appareil pour pouvoir y introduire un nouvelle bande consommable.

## Revendications

1. Appareil distributeur nécessitant pour fonctionner l'emploi d'éléments consommables se présentant sous la forme de bande (34), chacune de ces bandes comprenant dans le sens longitudinal plusieurs zones d'utilisation successives (Z1 à Z7) destinées à être séparées de la bande dès après leur utilisation, ledit appareil comprenant:
- un boîtier (20) définissant un couloir de circulation (29) pour lesdites bandes, ledit couloir présentant, compte tenu du sens d'introduction desdites bandes, une extrémité amont (AM) et une extrémité aval (AV),
- un mécanisme d'avance (30) pour faire sortir ladite bande (34) du couloir de circulation (29) selon un mouvement pas-à-pas (F1) après son introduction dans celui-ci par un utilisateur,
- une unité coulissante (31) montée mobile dans ledit couloir (29), couplée fonctionnellement audit mécanisme d'avance (30) et destinée à être accouplée à l'extrémité aval (AV) de ladite bande (34) lors de son introduction par l'utilisateur, et
- des moyens de couplage (41) portés par ladite unité coulissante (31) et agencés pour assurer le couplage entre celle-ci et ladite bande (34), ledit appareil distributeur étant caractérisé en ce que ladite unité coulissante (31) comprend un curseur (35) monté coulissant dans ledit couloir de circulation (29) et muni d'un organe d'entraînement (72), ledit mécanisme d'avance (30) comprenant des moyens d'encliquetage (54, 57, 62a à 67) destinés à coopérer avec ledit organe d'entraînement (72) pour assurer ledit couplage et permettre l'avance pas-à-pas dudit curseur (35), et en ce que lesdits moyens d'encliquetage (54, 57, 62a à 67) sont couplés à un bouton d'actionnement (23) monté mobile dans ledit boîtier (20) pour permettre la commande dudit mouvement pas-à-pas.

2. Appareil distributeur selon la revendication 1, caractérisé en ce que lesdits moyens d'encliquetage comprennent une barre d'encliquetage (57) montée dans ledit boîtier parallèlement audit couloir de circulation (29) de manière à pouvoir exécuter un mouvement orbital (F5 à F8) dans son propre plan, ladite barre d'encliquetage présentant des moyens pour, au cours de chaque mouvement orbital correspondant à l'avance d'un pas dudit curseur (35), venir en prise avec ledit organe d'entraînement (72) de celui-ci.

3. Appareil distributeur selon la revendication 2, caractérisé en ce que ledit mouvement orbital (F5 à F8) présente un tracé rectangulaire.

4. Appareil distributeur selon l'une quelconque des revendications 2 et 3, caractérisé en ce que ladite barre d'encliquetage (57) comprend une denture à dents de scie (59) dont les dents (60) sont destinées à venir en prise avec ledit organe d'entraînement (72).

5. Appareil distributeur selon l'une quelconque des revendications 2, 3 et 4, caractérisé en ce que ladite barre d'encliquetage (57) présente au moins une découpe (63a, 63b) dans laquelle est engagé un organe suiveur (67) fixe par rapport audit boîtier (20) pour définir avec ladite découpe (63a, 63b) ledit mouvement orbital (F5 à F8).

6. Appareil distributeur suivant l'une quelconque des revendications 2 à 5, caractérisé en ce qu'il est prévu des moyens élastiques de rappel (54) sollicitant ladite barre d'encliquetage (57) dans le sens opposé au sens d'introduction de ladite barre consommable (34) dans ledit couloir de circulation (29).

7. Appareil distributeur suivant l'une quelconque des revendications 1 à 6, caractérisé en ce que des crans d'indexation (44) sont prévus dans ledit couloir de circulation (29) au pas dudit mouvement pas-à-pas et en ce que ledit organe d'entraînement (72) est agencé pour venir en prise avec lesdits crans d'indexage (44) pour fixer chaque position dudit curseur (35).
